Europäisches Patentamt

European Patent Office

Office européen des brevets

(11) Publication number: **0 213 918**
**B1**

(12) **EUROPEAN PATENT SPECIFICATION**

(45) Date of publication of patent specification: **28.11.90**

(21) Application number: **86306577.7**

(22) Date of filing: **26.08.86**

(51) Int. Cl.⁵: **A 61 K 9/26,** A 61 K 9/18, A 61 K 9/22

(54) A process for producing a medical resin composition.

(30) Priority: **23.08.85 JP 186318/85**

(43) Date of publication of application:
**11.03.87 Bulletin 87/11**

(45) Publication of the grant of the patent:
**28.11.90 Bulletin 90/48**

(84) Designated Contracting States:
**DE FR GB**

(56) References cited:
**EP-A-0 136 916**
**EP-A-0 163 178**
**GB-A-1 376 277**
**US-A-3 921 636**
**US-A-4 379 038**

(73) Proprietor: **SUMITOMO ELECTRIC INDUSTRIES LIMITED**
**No. 15, Kitahama 5-chome Higashi-ku**
**Osaka-shi Osaka 541 (JP)**

(72) Inventor: **Takahashi, Hideo c/o Itami Works Sumitomo Electric Ind. Ltd. 1-1, Koyakita 1-chome**
**Itami-shi Hyogo (JP)**
Inventor: **Iwata, Kouichi c/o Itami Works Sumitomo Electric Ind. Ltd. 1-1, Koyakita 1-chome**
**Itami-shi Hyogo (JP)**

(74) Representative: **Pearce, Anthony Richmond et al**
**MARKS & CLERK Alpha Tower Suffolk Street Queensway Birmingham B1 1TT (GB)**

Courier Press, Leamington Spa, England.

## Description

Field of the invention

The present invention relates to a process for producing a medical resin composition having a sustained drug release capability over a prolonged period of time.

Background of the invention

Methods have recently been proposed wherein a drug may be combined with a resin to provide it with the efficacy of the drug. For example, Unexamined Published Japanese Patent Application No. 65009/1984 corresponding to U.S. Patent 4,555,398 describes a method of preparing a long-lasting vasodilator by blending the drug with a resin. In this method, however, the vasodilator is merely added to the resin and dispersed therein. As a result, it is difficult to control the release rate of the drug and its sustained release is limited to a fairly short period of time.

Japanese Patent Publication No. 42603/1981 describes a method wherein ionic bonds are formed between an anionic anticoagulant with cationic groups in a polymer such that the coagulant will exhibit its efficacy over a prolonged period of time. The composition prepared by this method has the desired long-lasting efficacy but its cost is high because of the complexity of the steps of introducing cationic groups into the polymer matrix and of forming ionic bonds with the anticoagulant.

In Unexamined Published Japanese Patent Application No. 76562/1985 corresponding to U.S. Patent Application Serial No. 657,437 filed October 3, 1984 describes a slow-release anticoagulant can be obtained in comparatively simple steps by blending a resin with a solution of an anticoagulant and an organic or inorganic powder serving as an adsorption carrier for the solution. However, this method allows the solution of anticoagulant to be adsorbed only on the surface layer of the carrier powder, and the amount of the anticoagulant solution that can be incorporated in the composition is fairly small. If too much of the anticoagulant solution is added, macrodomains of liquid particles form in the composition and cause an undesirably excessive release of the drug solution from the composition in the initial period of its use.

Summary of the invention

An object of the present invention is to develop a process of producing a resin composition which incorporates a large amount of drug solution and yet permits the drug solution to be released slowly over a prolonged period of time.

According to the present invention, there is provided a process for producing a medical resin composition which comprises the steps of: (A) preparing a slurry or suspension by adding and mixing (i) a porous powder with (ii) a mixed solution composed of solvent A which is capable of dissolving a drug at the final stage, solvent B which is miscible with solvent A and capable of dissolving a drug and boils at a lower temperature than solvent A, and (iii) the drug; (B) removing solvent B from the slurry or suspension by vacuum drying so that a drug solution composed of the drug and solvent A is incorporated within the pores of the porous powder; and (C) dispersing said porous powder uniformly in a resin.

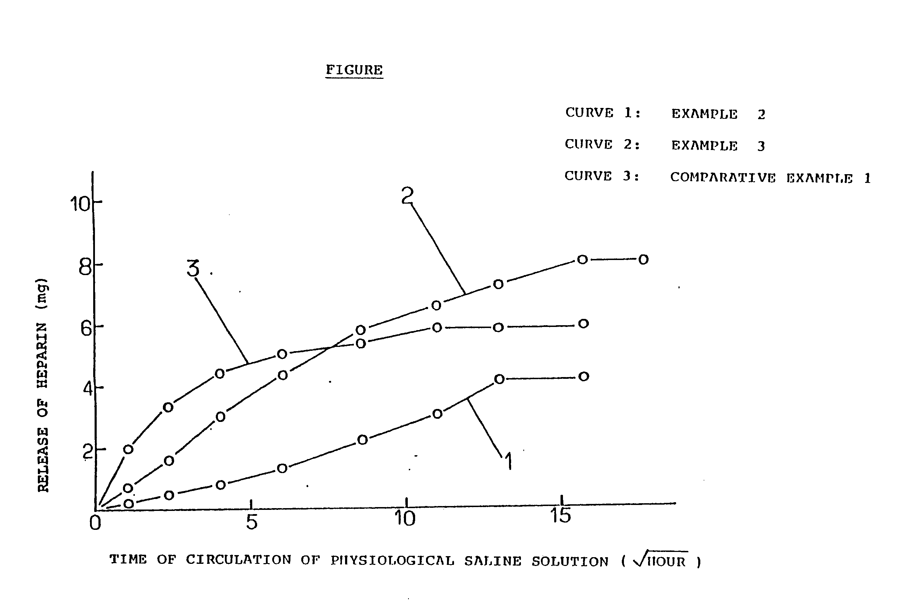

Brief description of the drawing

The Figure is a graph showing the relation between the time of circulation of a physiological saline solution (Hour) and the release of the drug, heparin (mg).

Detailed description of the invention

In the present invention a greater amount of the drug solution can be uniformly dispersed in the resin by adding to the resin both the drug solution and a porous powder which has the drug solution incorporated in the small pores in the powder.

The medical resin composition prepared by the process of the present invention may be formed into any shape by standard forming techniques and may be used as tablets for burial in the human body or as tubes, valves or membranes for transfusion purposes. When in use, the drug solution absorbed on the surface of the porous powder is first released from the composition, then the drug solution in the small pores in the powder is slowly released, thereby allowing the composition to release the drug solution in a sustained manner over a time period much longer than has been previously attainable.

Examples of the porous powder can be used in the present invention include activated carbon, silica gel, synthetic zeolite and porous polystyrene. Any other materials may be used in the present invention so long as they are powders having small pores which communicate with the surface of individual particles. Porous powders having large specific surface areas are desirable because of their high adsorbing capability. The pore diameter and void volume of the porous powder can be selected appropriately. For example, silica gel porous powder preferably has a pore diameter of about $2\times10^{-9}$ m to about $3\times10^{-8}$ m (about 20 to about 300 angstroms) and a void volume of about 0.4 to about 1.6 ml/g. Porous activated carbon powder preferably has a pore diameter of about $2\times10^{-9}$ m to about $2\times10^{-8}$ m (about 20 to about 200 angstroms) and a void volume of about 0.7 ml/g. Preferred void volume of porous synthetic zeolite is about 0.3 ml/g. Porous polystryrene preferably has a pore diameter of about $3\times10^{-8}$ m to about $1.9\times10^{-7}$ m (about 300 to about 1,900 angstroms).

The synthetic resin used in the present invention may be selected from a list of polymers that have the strength and elasticity required for medical polymers, exhibit corrosion resistance in the human body, cause no hazard to human health, and which can be formed or worked into final shapes at temperatures below the decomposition point of a specific drug. Thereafter, the synthetic resin for use in the present invention can be selected from a broad range of commercial polymers including plastics, elastomers, rubbers and casting resins.

Examples of the synthetic resin which can be used in the present invention include ethylene/α-olefin copolymers as described in, for example, Unexamined Published Japanese Patent Application No. 82734/83, ethylene/vinyl acetate copolymers as described in, for example, Unexamined Published Japanese Patent Application No. 29338/83, vinyl chloride resins, etc. Of these, ethylene/α-olefin copolymers are preferred. Specific examples of α-olefin which can be used include 1-butene, 1-pentene, 1-hexene, 4-methyl-1-pentene, 1-octene, 1-decene, 1-tetradecene, 1-octadecene, etc. These monomers can be used singly or in combination. It is preferred to use α-olefins having 6 to 18 carbon atoms in view of high impact strength and high resistance to stress cracking.

Examples of the drug that may be used in the present invention include anticoagulants such as heparin and alginic acid sulfate; bactericides such as chlorohexidine; thrombolytic agents such as urokinase; anti-tumor agents such as adriamycin; and hormones. These drugs may be dissolved in appropriate solvents having higher boiling points than the temperature at which the resin matrix is to be worked, thereby preparing a drug solution suitable for use in the present invention.

In previously proposed methods of incorporating the drug solution within the small pores in the porous powder, a liquid is usually incorporated within small pores by either impregnating the liquid in small pores under vacuum or replacing the air in the small pores with the liquid under pressure. However, the powders obtained by these methods are wetted with the liquid and present a considerable degree of stickiness that causes much inconvenience in subsequent handling. The process of the present invention makes it possible to provide a porous powder which contains a liquid in small pores and which yet retains a high degree of fluidity. In the process of the present invention, solvent A which is capable of dissolving the drug at the final stage, and solvent B which is miscible with solvent A and capable of dissolving the drug and which boils at a lower temperature than solvent A are mixed with the drug, a porous powder is added to the mixed solution and mixed to form a slurry or suspension, and solvent B is removed from the resulting mixture by drying under vacuum. The slurry or suspension, when it is freed of solvent B, becomes a solid cake which will readily form a highly fluid powder upon exertion of a light shear stress. The ability of solvent A to dissolve the drug refers to its ability at the final stage after solvent B is removed, not when the drug is simply added to solvent A.

It is essential for the purpose of the present invention that solvent B be miscible with solvent A and capable of dissolving the drug and that it boils at a lower temperature than solvent A. Desirably, solvent B is less viscous than solvent A in order to prepare a mixed solution of a lower viscosity that will readily enter into the small pores in the porous powder.

Various combinations of solvents can be used in the process of the present invention. For example, a combination of glycerol as solvent A and water as solvent B is preferred for chlorohexidine and sodium heparinate.

The amount of the drug solution (i.e., drug and solvent A) must be smaller than total void volume of the porous powder. When the amount of the drug solution exceeds the total void volume of the porous powder, finally obtained powder are undesirably sticky, resulting in reduced workability. For example, when porous silica gel as porous powder and glycerol having dissolved therein sodium heparinate as drug solution are used the amount of the drug solution is preferably about 0.7 to about 1.1 ml, more preferably about 0.9 ml, per gram of silica gel. When it exceeds about 1.1 ml the porous powder after dehydration tends to be sticky while the resulting porous powder has acceptable flowability but the drug solution is difficult to be released when the resulting powder is dispersed in a resin.

The concentration of the drug in the drug solution can be selected appropriately. For example, in the case where sodium heparinate and glycerol are used, the concentration of heparin is preferably about 10 to about 40 wt%, more preferably about 20 wt%. When it is below about 10 wt% it is necessary to add a large amount of drug solution and of porous powder in order to increase the content of the drug contained in the medical resin composition. On the other hand, when it exceeds about 40 wt% secondary agglomeration of powder impregnated with the drug solution tends to occur and it is difficult to disperse the resulting porous powder in a resin uniformly.

Solvent B is used in such an amount that a uniform slurry is obtained and each individual particle of the porous powder is wetted. If the amount of solvent B is too large it takes a long period of time for removing the solvent. Usually, it is preferred to use solvent B in an amount of about 100 to 500 ml per 100 g of the porous powder. Any conventional mixer/stirrer can be employed in preparing a slurry or suspension by adding the porous powder to the mixed solution composed of solvents A and B and the drug. Usually, preparation of the slurry or suspension can be carried out at room temperature. In order to obtain a uniform dispersion, it is desirable to use a dissolver. For example, a stirrer is used to prepare a mixed solution of solvent A, solvent B and a drug. The porous powder is added to this mixed solution and is stirred using a dissolver at a low speed (e.g., 500 rpm) for about 2 to 3 minutes to wet the porous powder and then at a high speed (about 2,000 rpm) for about 30 minutes to obtain a uniform slurry.

The following examples are provided for the purpose of further illustrating the present invention.

Example 1

A homogeneous mixture was prepared from 10 g of a sodium heparinate powder (anticoagulant), 500 g of water (b.p. 100°C) and 40 g of glycerol (b.p. 290°C). A suspension was formed by dispersing 25 g of silica gel (average particle size; 3 µm; void volume; 1.6 ml/g) to the mixed solution. The suspension was dehydrated by drying at 60°C under vacuum of 50 mm Hg for 6 hours and 5 mm Hg for 6 hours. The resulting solid cake was placed in a mortar and a light shear stress was applied with a pestle to form a non-sticky and highly fluid fine powder. Analysis with a thermobalance showed that the powder contained 33 wt% of a solid residue at 600°C, indicating that the powder contained the necessary amount of the drug (anticoagulant).

Example 2

The powder obtained in Example 1 was added to and mixed with an ethylene/α-olefin copolymer resin in such a manner that the powder content was 20 wt%. The mixture was fed into a screw-type kneader where it was melted and kneaded at 100°C and extruded into a tube having an inside diameter of 3 mm and an outside diameter of 4 mm.

The tube was cut to a length of 30 cm and a physiological saline solution was circulated through the cut tube at a rate of 5 ml/min. The time-dependent change of the release of the sodium heparinate into the physiological saline solution was determined on the basis of color reaction between toluidine blue and sodium heparinate. The results are shown in the Figure by curve 1.

Example 3

The powder (20 wt%) obtained in Example 1 and 10 wt% of a glycerol solution containing 20 wt% of sodium heparinate were added to and mixed with an ethylene/α-olefin copolymer resin. The mixture was extruded as in Example 2 into a tube having an inside diameter of 3 mm and an outside diameter of 4 mm. The time-dependent change of the heparin release from the tube was determined as in Example 2, and the results are shown in the Figure by curve 2.

Comparative Example 1

A glycerol solution (13.3 wt%) containing 20 wt% of sodium heparinate was added to and mixed with an ethylene/α-olefin copolymer resin, together with 6.7 wt% of silica gel (average particle size; 3 µm; void volume; 1.6 ml/g). A tube was extruded from the mixture and subjected to a release test as in Example 2. The results are shown in the Figure by curve 3.

As is evident from the Figure, the medical resin compositions prepared in accordance with the present invention did not experience any excessive drug release at the initial stage of use and exhibited sustained drug release over a prolonged period of time.

Claims

1. A process for producing a medical resin composition which comprises the steps of: (A) preparing a slurry or suspension by adding and mixing (i) a porous powder with (ii) a mixed solution composed of solvent A which is capable of dissolving a drug at the final stage, solvent B which is miscible with solvent A and capable of dissolving a drug and boils at a lower temperature than solvent A, and (iii) the drug; (B) removing solvent B from the slurry or suspension by vacuum drying so that a drug solution composed of the drug and solvent A is incorporated within the pores of the porous powder; and (C) dispersing said porous powder uniformly in a resin.

2. A process as claimed in Claim 1, wherein in step (C) a solution of said drug is also uniformly dispersed in the resin.

3. A process as claimed in Claim 1 or 2, wherein said porous powder is selected from the group consisting of activated carbon, silica gel, synthetic zeolite and porous polystyrene.

4. A process as claimed in any preceding Claim, wherein said drug is selected from the group consisting of anticoagulants, bactericides, thrombolytic agents, anti-tumor agents and hormones.

5. A process as claimed in any preceding claim, wherein solvent B is less viscous than solvent A.

6. A process as claimed in Claim 1, wherein the drug is chlorohexidine or sodium heparinate, solvent A is glycerol, and solvent B is water.

Patentansprüche

1. Verfahren zum Herstellen einer medizinichen Harzzusammensetzung, umfassend die folgenden Schritte: (A) Zubereiten einer Schlempe oder Suspension durch Hinzufügen und Mischen (i) eines porösen Pulvers zu bzw. mit (ii) einer gemischten Lösung aus einem Lösungsmittel A, das in der Lage ist, ein Medikament im Endzustand aufzulösen, einem Lösungsmittel B, das mit dem Lösungsmittel A vermischbar ist und in der Lage ist, ein Medikament aufzulösen und das bei einer niedrigeren Temperatur als das Lösungsmittel A siedet und (iii) dem Medikament; (B) Entfernen des Lösungsmittels B aus der Schlempe oder Suspension durch Vakuumtrocknung derart, daß eine Medikamentenlösung aus dem Medikament und dem Lösungsmittel A in die Poren des porösen Pulvers eingebaut wird; und (C) gleichförmiges Dispergieren des porösen Pulvers in ein Harz.

2. Verfahren nach Anspruch 1, bei dem im Schritt (C) eine Lösung aus dem Medikament ebenfalls gleichmäßig in dem Harz dispergiert wird.

3. Verfahren nach Anspruch 1 oder 2, bei dem das poröse Pulver aus der Gruppe ausgewählt wird, die Aktivkohle, Silicagel, synthetisches Zeolith und poröses Polystyrol umfaßt.

4. Verfahren nach einem der vorhergehenden Ansprüche, bei dem das Medikament aus der Gruppe ausgewählt wird, die Antikoagulansien, Bakterizide, thromolytische Mittel, Antitumormittel und Hormone umfaßt.

5. Verfahren nach einem der vorhergehenden Ansprüche, bei dem das Lösungsmittel B weniger viskos ist, als das Lösungsmittel A.

6. Verfahren nach Anspruch 1, bei dem das Medikament Chlorhexidin oder Natriumheparinat, das Lösungsmittel A Glyptal und das Lösungsmittel B Wasser ist.

**Revendications**

1. Un procédé pour fabriquer une composition pharmaceutique de résine qui comprend les étapes suivantes: (A) préparation d'une bouille ou suspension en ajoutant et mélangeant (i) une poudre poreuse avec (ii) une solution mélangée composée d'un solvant A capable de dissoudre un médicament dans le stade final, d'un solvant B qui est miscible avec le solvant A et capable de dissoudre un médicament et qui bout à une température plus basse que le solvant A et (iii) le médicament; (B) séparation du solvant B de la bouillie ou suspension par séchage sous vide de manière à incorporer dans les pores de la poudre poreuse une solution de médicament composée du médicament et du solvant A; et (C) dispersion uniforme de ladite poudre poreuse dans une résine.

2. Un procédé selon la revendication 1, dans lequel dans l'étape (C) une solution dudit médicament est également dispersée uniformément dans la résine.

3. Un procédé selon la revendication 1 ou 2, dans lequel ladite poudre poreuse est choisie parmi le charbon actif, le gel de silica, la zéolite synthétique et le polystyrène poreux.

4. Un procédé selon l'une quelconque des revendications précédentes, dans lequel ledit médicament est choisi parmi les anticoagulants, les bactéricides, les agents thrombolytiques, les agents antitumoraux et les hormones.

5. Un procédé selon l'une quelconque des revendications précédentes, dans lequel le solvant B est moins visqueux que le solvant A.

6. Un procédé selon la revendication 1, dans lequel le médicament est la chlorohexidine ou l'héparinate de sodium, le solvant A est le glycérol et le solvant B est l'eau.

FIGURE

CURVE 1:   EXAMPLE  2

CURVE 2:   EXAMPLE  3

CURVE 3:   COMPARATIVE EXAMPLE 1

TIME OF CIRCULATION OF PHYSIOLOGICAL SALINE SOLUTION ( $\sqrt{\text{HOUR}}$ )

EP 0 213 918 B1